(19) Europäisches Patentamt
European Patent Office
Office européen des brevets

(11) EP 1 334 204 B1

(12) FASCICULE DE BREVET EUROPEEN

(45) Date de publication et mention de la délivrance du brevet:
**31.10.2007 Bulletin 2007/44**

(21) Numéro de dépôt: **01996620.9**

(22) Date de dépôt: **16.11.2001**

(51) Int Cl.:
***C12Q 1/04** (2006.01)* ***C12Q 1/10** (2006.01)*
***C12N 1/20** (2006.01)* ***C12Q 1/44** (2006.01)*

(86) Numéro de dépôt international:
**PCT/FR2001/003610**

(87) Numéro de publication internationale:
**WO 2002/040704 (23.05.2002 Gazette 2002/21)**

# (54) PROCEDE ET MILIEU DE DETECTION/IDENTIFICATION DE MICROORGANISMES A ACTIVITE ESTERASIQUE

VERFAHREN UND MEDIUM ZUM NACHWEISEN/IDENTIFIZIEREN VON MIKROORGANISMEN MIT ESTERASEAKTIVITÄT

METHOD AND MEDIUM FOR DETECTING/IDENTIFYING MICRO-ORGANISMS WITH ESTERASE ACTIVITY

(84) Etats contractants désignés:
**AT BE CH CY DE DK ES FI FR GB GR IE IT LI LU MC NL PT SE TR**

(30) Priorité: **17.11.2000 FR 0014881**

(43) Date de publication de la demande:
**13.08.2003 Bulletin 2003/33**

(73) Titulaire: **BIO MERIEUX**
**69280 Marcy l'Etoile (FR)**

(72) Inventeur: **ROGER-DALBERT, Céline**
**F-01150 VAULX-EN-BUGEY (FR)**

(74) Mandataire: **Fleurance, Raphaël et al**
**Cabinet Plasseraud**
**52 rue de la Victoire**
**75440 Paris Cedex 09 (FR)**

(56) Documents cités:
**WO-A-99/41409** **FR-A- 2 697 028**

- **COOKE VENITIA M ET AL: "A novel chromogenic ester agar medium for detection of salmonellae." APPLIED AND ENVIRONMENTAL MICROBIOLOGY, vol. 65, no. 2, février 1999 (1999-02), pages 807-812, XP002177548 ISSN: 0099-2240**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 334 204 B1

Printed by Jouve, 75001 PARIS (FR)

## Description

Le domaine de l'invention est celui de l'analyse microbiologique par voie biochimique, et en particulier de la détection et de l'identification de souches de micro-organismes par ensemencement de milieux réactionnels, en particulier des milieux nutritifs. Ces derniers comprennent des substrats chromogènes ou fluorogènes susceptibles de réagir avec les enzymes spécifiques des souches recherchées, en produisant une coloration ou une fluorescence de chaque colonie concernée.

On s'intéresse plus particulièrement dans le cadre du présent exposé à la détection/identification de micro-organismes à activité estérasique, et plus spécialement de bactéries du genre *Salmonella.*

Ce genre *Salmonella* est le plus important de la famille des entérobactéries responsables chez l'homme de diverses infections (fièvre typhoïde, intoxication alimentaire). Les salmonelles possèdent des estérases non spécifiques capables d'hydrolyser des substrats synthétiques chromogènes, par exemple indigogéniques ou des substrats fluorogènes.

La détection/identification de salmonelles, et plus généralement de micro-organismes à activité estérasique est classiquement réalisée sur des milieux gélosés ou liquides d'isolement, qui permettent la détection/identification des colonies suspectes de micro-organismes à activité estérase, notamment les salmonelles. L'ensemencement de tels milieux s'opère par trempage dudit milieu dans l'échantillon analysé ou par mise en contact de l'échantillon avec le milieu.

Les micro-organismes à activité estérasique, en particulier les salmonelles, possèdent dans leur patrimoine enzymatique des estérases qui clivent les liaisons ester des substrats présents dans le milieu et qui libèrent ainsi la partie chromogène ou fluorogène activée desdits substrats. Il en résulte une coloration ou une fluorescence qui révèle l'hydrolyse, et donc la présence de micro-organismes ou de colonies de micro-organismes cibles.

Pour pouvoir réaliser des tests de routine à grande envergure, il est nécessaire que les milieux de détection/identification soient stables et permettent de simplifier au maximum les procédés de détection/identification correspondants, en limitant les manipulations. En outre, il importe que les procédés offrent une haute sensibilité (intensité de coloration ou de fluorescence), ainsi qu'une spécificité de premier ordre. La vitesse de révélation des colonies suspectes est également un paramètre fondamental de ces types de milieux et procédés de détection/identification de micro-organismes à activité estérasique.

Or, il est connu que les substrats d'estérase posent des problèmes de compatibilité avec les milieux de culture pour micro-organismes à activité estérase. De plus, de tels substrats d'estérase ne sont pas stables dans le temps, ce qui induit une diminution de la sensibilité vis-à-vis de l'activité estérase avec l'allongement du temps de conservation.

De plus, il serait également appréciable de disposer d'additifs, d'une part, qui confèrent à ce genre de milieux, une translucidité à l'état non ensemencé (lecture croissance non perturbée) ainsi qu'une sélectivité vis à vis de certaines souches et, d'autre part, qui promeuvent l'activité enzymatique utile comme révélateur (coloration plus franche).

Dans ce contexte, le brevet FR-2 697 028 divulgue un milieu de culture pour la mise en évidence de salmonelles comprenant un substrat d'estérase chromogène constitué par un ester de l'acide de caprylique avec un reste indole (5-Bromo-4-chloro-3-indolyl-caprylate), ainsi qu'un détergent choisi parmi les sels biliaires (Désoxycholate de Sodium). Ce chromogène et ce sel biliaire sont contenus dans un milieu nutritif permettant la croissance des salmonelles. Selon l'enseignement du FR 2 697 028, le sel biliaire est ajouté directement au milieu sélectif dans lequel se trouve déjà inclus le substrat d'estérase.

Un inconvénient lié à l'emploi de sels biliaires tient au fait que ces derniers sont des matières premières d'origine animale, ce qui sous-tend une certaine variabilité de la qualité.

De plus, les résultats en termes d'activité biologique sont perfectibles.

Par ailleurs, ce milieu de culture n'offre pas toutes les garanties souhaitables en terme de stabilité du substrat d'estérase. En effet, il s'avère que ce dernier n'est pas complètement miscible avec le milieu de culture. Il est clair que cela nuit à la qualité des résultats obtenus sur le plan de la sensibilité et de la rapidité.

Il doit également être noté que le milieu de culture selon le FR 2 697 028 se présente sous forme de poudre. Cela oblige l'utilisateur à exécuter une opération de reconstitution préalable du milieu liquide ou gélifié. Cette contrainte résulte du manque de stabilité des substrats d'estérase mis en oeuvre.

De surcroît, le milieu gélosé préparé selon l'enseignement du FR 2 697 028 n'est pas translucide. Ceci risque de compromettre la lecture des colorations associées aux éventuelles colonies de bactéries cibles.

Enfin, il est indiqué page 3, lignes 17-23 de ce document que les détergents anioniques commercialisés sous la marque Tergitol® par UNION CARBIDE (alkyl-sulfate de Na) ne permettent pas de mettre en évidence une coloration.

La demande de brevet PCT WO-92/17607 concerne un milieu de détection pour salmonelles contenant du TERGITOL® 4 (sulfate hydrogéné de 7-éthyl-2-méthyl-4-undécanoate ou son sel de sodium). Cet additif est censé améliorer la sélectivité du milieu de détection vis-à-vis des germes autres que les salmonelles (inhibition croissance *Proteus spp* notamment). La concentration en TERGITOL® 4 peut varier de 2 à 30 ml/l dans le milieu de culture à base de gélose agar-xylose-lysine. Selon ce document, la détection de salmonelles repose en partie sur un principe de croissance sélective par compétition. En outre, ce milieu ne contient pas de substrats d'estérase chromogènes ou

fluorogènes.

La demande PCT WO-99/41409 concerne des substrats d'esterase chromogènes pour la détection de salmonelles. Il est proposé selon ce document de mettre en oeuvre un composé chromogène qui réagit avec une estérase/lipase spécifique du genre *Salmonella* et ayant une affinité pour les esters d'acide gras en C8. Le composé chromogène dont il s'agit, comprend un anion et un cation de formule : [4-[2-(4-octanoyloxy-3,5-diméthoxyphényl)-vinyl]-quinolinium-1-(propan-3-yle-acide carboxylique]$^+$, $^-$[bromure ou chlorure].

Plus précisément, les substrats mis en oeuvre sont des esters en C8, par exemple, du cation carboxylate. En outre, le procédé selon le WO 99/41409 décrit la mise en oeuvre d'ester de Sorbitan et d'acides gras (de préférence, acide monolaurique : TWEEN® 20). Ces produits sont employés à titre de détergent à raison de 2 g par litre de milieu pour améliorer la transparence du milieu avant ensemencement et la coloration des colonies cibles pour le substrat d'estérase chromogène spécifique susvisé. Le Triton® X100 et le Niaproof® 8 (=Tergitol® 8) font partie des détergents cités dans ce document, parmi une pluralité d'autres détergents. Aucun exemple de milieu contenant le Triton® X100 ou le Niaproof® 8 (=Tergitol® 8) n'est donné.

En tout état de cause, les moyens divulgués dans le WO 99/41409 ne sont pas présentés comme procurant une amélioration de la sélectivité, ni de la spécificité des substrats enzymatiques contenus dans le milieu de détection.

Dans un tel environnement technique, l'un des objectifs essentiels de la présente invention est de fournir un milieu de détection/identification de micro-organismes à activité estérasique, qui soit constitué de telle sorte que la sensibilité de l'analyse soit optimisée, c'est-à-dire que l'intensité de la coloration révélant la présence de micro-organismes cibles soit la plus forte possible (promotion de l'activité estérase).

Un autre objectif essentiel de la présente invention est de fournir un milieu de détection/identification de micro-organismes à activité estérasique, qui soit parfaitement translucide avant ensemencement par l'échantillon à analyser.

Un autre objectif essentiel de l'invention est de fournir un milieu de détection/identification de micro-organismes à activité estérasique, qui comporte un substrat d'estérase chromogène ou fluorogène et qui soit stable à la conservation (intensité des colorations de révélation maintenue à un niveau maximum au moins pendant plusieurs semaines).

Un autre objectif essentiel de l'invention est de fournir un milieu de détection/identification de micro-organismes à activité estérasique, qui ne se présente pas sous forme de poudre sèche à régénérer avec un liquide pour reconstituer un milieu liquide ou gélifié, mais qui existe directement sous des formes prêtes à l'emploi.

Un autre objectif essentiel de l'invention est de fournir un milieu de détection/identification de micro-organismes à activité estérasique, qui soit économique, notamment du fait qu'il comprend des quantités réduites de substrat d'estérase chromogène ou fluorogène, lequel a la particularité d'être coûteux.

Un autre objectif essentiel de l'invention est de fournir un milieu de détection/identification de micro-organismes à activité estérasique, en particulier d'entérobactéries gram- e.g. salmonelles, de bactéries gram$^+$ ou de levures.

Un autre objectif essentiel de l'invention est de fournir un procédé d'obtention du milieu de détection/identification sus visé qui soit simple à mettre en oeuvre et économique.

Un autre objectif essentiel de la présente invention est de fournir un procédé de détection/identification de souches à activité estérasique qui soit facile à mettre en oeuvre (tests de routine), qui soit économique (quantité de réactif, manipulation, main d'oeuvre, vitesse...) qui soit fiable, sensible, spécifique et reproductible.

Ces objectifs, parmi d'autres, sont atteints par la présente invention qui concerne tout d'abord un milieu de détection/identification de micro-organismes à activité estérasique, du type de ceux comprenant notamment un milieu réactionnel (M) (en particulier un milieu de culture) et au moins un substrat d'estérase chromogène ou fluorogène (S) ; cette détection/identification reposant essentiellement sur la mise en évidence de l'activité estérase,
caractérisé en ce que :

- ✧ il est sous forme stable liquide ou gel prêt à l'emploi ;
- ✧ il comporte :
  - o -A- au moins un agent solubilisant et stabilisant choisi dans le groupe comprenant :
    - o les Esters de Sorbitan et d'Acide(s) Gras (ESAG),
    - o les sels biliaires,
    - o et leurs mélanges;
  - o -B- au moins un activateur sélectif sélectionné dans le groupe comprenant:
    - ■ les composés de formule (I) :

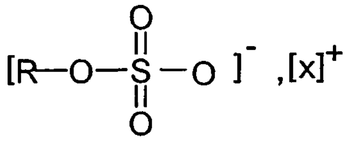

**(I)**

dans laquelle :

- R est un alkyle linéaire ou ramifié, de préférence en C1-C30;
- X est un halogène, de préference Na;

✧ les dérivés polyéthers non ioniques, de préférence ceux de formule**(II):**

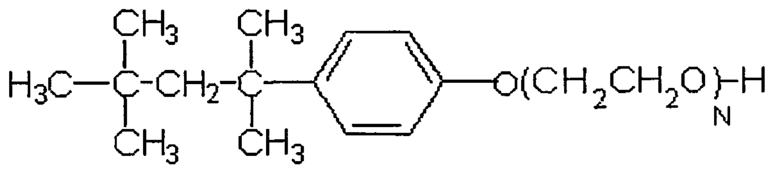

N est un nombre entier compris entre 2 et 15 (en moyenne = 10);

✧ et leurs mélanges;

o -C- et éventuellement au moins un Solvant.

Le milieu selon l'invention présente l'avantage d'offrir des colorations particulièrement intenses des colonies de micro-organismes cibles à activité estérase. Cela facilite grandement la lecture et l'interprétation des résultats.

Il est également intéressant de noter que ce milieu ne favorise pas la croissance des micro-organismes n'ayant pas une activité estérase. Cette spécificité est tout à fait bénéfique pour l'analyse.

Il est également avantageux que ce milieu soit prêt à l'emploi et se présente sous forme liquide ou semi-liquide (gel) sans que cela ne pose de problème quant à la stabilité. En effet, les substrats d'estérase présents dans ce milieu et pourtant connus pour leur tendance à la dégradation relativement rapide, sont stabilisés grâce à la présence des additifs A, B et éventuellement C.

Ces additifs induisent un autre avantage qui est de permettre une excellente dissolution des substrats d'estérase traditionnellement récalcitrants à la mise en solution. Cela permet d'obtenir des milieux de détection/identification translucides avant l'ensemencement. La lecture des résultats colorés ou fluorescents s'en trouve ainsi plus aisée.

Enfin, ces additifs A, B et éventuellement C sont peu onéreux et permettent une simplification de la mise en oeuvre et une diminution de la quantité des substrats d'estérase utilisée (stabilisation). Il en résulte un gain économique certain.

Il est du mérite des inventeurs d'avoir sélectionné cette classe particulière d'activateurs spécifiques B et de les avoir associés aux agents solubilisants (A) (e.g. les ESAG ou les sels biliaires). En effet, ils procurent de manière surprenante et inattendue des propriétés, d'activité biologique, de sensibilité, de fiabilité, de spécificité, de stabilité, de sélectivité et de translucidité tout à fait bienvenues dans l'analyse microbiologique par voie biochimique selon l'invention.

Suivant une caractéristique remarquable de l'invention, le milieu de détection/identification qu'elle concerne est obtenu par mélange d'au moins une solution mère de substrat (S) dans le solvant (C) et d'agent solubilisant (A), ajouté à au moins une partie des constituants du milieu réactionnel (M) avec le promoteur B.

Il est en effet apparu important conformément à l'invention de solubiliser le substrat (S) d'estérase dans le solvant C, en présence d'un ou plusieurs agents (A). Ce mélange substrat (S) / (C) / (A) est ensuite incorporé dans au moins une partie du milieu M de culture, qui se présente, de préférence, sous forme gélifiée en surfusion. Il a pu être constaté que cette caractéristique opératoire permet une optimisation des résultats avantageux produits par les moyens selon l'invention. L'additif (B) est de préférence incorporé ensuite dans le milieu, comme cela sera détaillé ci-après.

Conformément à l'invention, le promoteur sélectif (B) est un produit de formule (**I**) dans laquelle R correspond à un radical tétradécyle (Tergitol® ou Niaproof® 4) ou à un radical 2-éthylhexyle (Tergitol® ou Niaproof® 8).

Le promoteur sélectif (B) peut également être un éther de mono-p-iso-octylphényle et de polyéthylène glycol de formule :

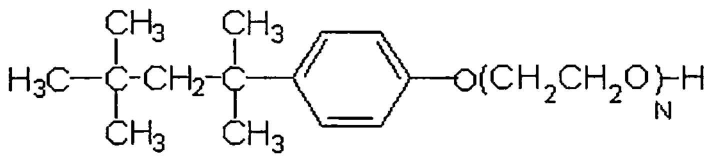

N est un nombre entier compris entre 2 et 15 (en moyenne = 10). Ces produits sont commercialisés sous les marques TRITON X-100®, TRITON X-114®, TRITON X-405®.
De préférence, l'ESAG (A) est sélectionné dans le groupe comprenant :

- ✧ Monolaurate de Sorbitan polyoxyéthylèné comprenant 20 motifs oxyde d'éthylène (O.E), -TWEEN® 20-;
- ✧ Monopalmitate de Sorbitan polyoxyéthylèné (20 O.E), -TWEEN® 40-;
- ✧ Monostéarate de Sorbitan polyoxyéthylèné (20 O.E), -TWEEN® 60-;
- ✧ Tristéarate de Sorbitan polyoxyéthylèné (20 O.E), -TWEEN® 65 -;
- ✧ Monooléate de Sorbitan polyoxyéthylèné (20 O.E), -TWEEN® 80-;
- ✧ Sesquioléate de Sorbitan polyoxyéthylèné (20 O.E), -TWEEN® 83-;
- ✧ Trioléate de Sorbitan polyoxyéthylèné (20 O.E), -TWEEN® 85-;
- ✧ et leurs mélanges.

Les produits sélectionnés ci-dessus (esters de Sorbitan) sont largement utilisés dans l'industrie alimentaire et dans l'industrie cosmétique comme émulsifiants non ioniques, mais jusqu'alors ils n'avaient jamais été employés dans des milieux de détection/identification microbiologiques, à titre de solubilisants vis-à-vis de substrats d'estérase, non susceptibles de réagir avec des lipases et différents des esters de sels de carboxylate.
Les "Hydrophile-Lipophile-Balances" (HLB) des ESAG sus-mentionnés sont respectivement : 8,6 ; 6,7 ; 4,7 ; 2,1 ; 4,3 ; 3,7 ; 1,8.
Dans le cas où les microorganismes recherchés sont des bactéries gram$^+$ et/ou des levures, la concentration en agent (B), exprimée en ml par litre de milieu (comprenant notamment: A, B, C, M, S) est telle que :

$$1.10^{-4} \leq [B] \leq 10$$

de préférence

$$1.10^{-3} \leq [B] \leq 5$$

et plus préférentiellement encore

$$1.10^{-2} \leq [B] \leq 2$$

Dans le cas où les micro-organismes recherchés sont des bactéries gram$^-$, la concentration en agent (B), exprimée en ml par litre de milieu (comprenant notamment: A, B, C, M, S) est telle que :

$$1.10^{-2} \leq [B] \leq 100$$

de préférence

$$1.10^{-1} \leq [B] \leq 30$$

et plus préférentiellement encore

$$1 \leq [B] \leq 25$$

L'utilisation de cette combinaison synergique (B)/(A)/ de préférence (C), facilite la mise en évidence de l'activité estérase dans les micro-organismes cibles. Elle permet d'améliorer la sélectivité du milieu et la détection de l'activité estérase des micro-organismes cibles estérase$^+$(par exemple *Salmonella),* sans nuire à l'expression d'autres activités enzymatiques qui pourraient être éventuellement utilisées pour la mise en évidence d'autres micro-organismes cibles considérées (β-galactosidase$^+$ ou β-glucosidase$^+$).

Sans vouloir être lié par la théorie, il est supposé que dans cette combinaison synergique, le composé (B) a une action qui favorise la pénétration des substrats d'estérase chromogènes ou fluorogènes ou l'excrétion d'enzymes au travers des membranes des cellules des micro-organismes cibles, par exemple des salmonelles, pour autant que ces derniers aient bien été solubilisés grâce à l'action de (A). Cela améliore l'accessibilité de ces substrats estérase$^+$. En conséquence, on peut diminuer la quantité de substrats mis en oeuvre et en tirer un avantage économique certain.

S'agissant du substrat d'estérase chromogène ou fluorogène (S), il est sélectionné avantageusement dans le groupe comportant :

- o les esters d'acide(s) gras et d'Indoxyle et dérivés, de préférence les esters d'acide(s) gras en C6-C11 et d'Indoxyle, et plus préférentiellement encore les esters d'acide(s) gras en C8-C9 et d'Indoxyle éventuellement halogénés;
- o les esters d'acide(s) gras et de Quinone/Anthraquinone et dérivés, de préférence les esters d'acide(s) gras en C6-C11 et d'Anthraquinone, et plus préférentiellement encore les esters d'acide(s) gras en C8-C9 et de Dihydroxyanthraquinone (Alizarine) ;
- o les esters d'acide(s) gras et d'Hydroxycoumarine et dérivés ;
- o les esters d'acide(s) gras et de Fluorescéine et dérivés ;
- o et leurs mélanges.

Comme exemples de substrats esters chromogènes dérivés d'indole, on peut citer le 5-Bromo-3-indolyl nonanoate, le 6-Chloro-3-indolyl nonanoate ou le 5-Bromo-3-indolyl décanoate.

A titre d'exemples de substrats d'estérase chromogènes dérivés de l'Anthraquinone, on peut citer, l'Alizarine et le 2-Alizarine octanoate.

Le solvant (C) est un auxiliaire de solubilisation du substrat d'estérase chromogène ou fluorogène. Il complète également l'action de l'agent solubilisant (A). Il s'agit donc de préférence d'un constituant du milieu selon l'invention.

Selon une disposition avantageuse de cette dernière, le solvant (C) est sélectionné dans le groupe comprenant :

- ■ les alcools, de préférence le Méthanol, l'Ethanol, le Méthoxyéthanol ;
- ■ les amides, de préférence le DiMéthylFormamide (DMF) ;
- ■ les solvants soufrés, de préférence le DiMéthylSulfOxyde (DMSO) ;
- ■ et leurs mélanges.

En pratique, les solvants utilisés sont le méthanol, le DMF et le DMSO.

De manière plus préférée encore, l'association (A) = TWEEN®20, (B) = NIAPROOF® 4 ou TERGITOL® 4 et solvant (C) =DMSO, apparaît comme étant tout à fait performante dans le milieu de détection/identification selon l'invention.

Sur le plan pondéral, il s'est avéré tout à fait avantageux, conformément à l'invention, que la proportion agent (A) : solvant (C) soit comprise entre 20: 80 et 80 : 20, de préférence entre 30:70 et 70:30, et plus préférentiellement encore corresponde à 40 : 60 ou à 60 : 40.

Toujours sur le plan quantitatif, il est préférable que la concentration en agent [A] dans le milieu soit définie comme suit (en % en poids) :

✧

$$0{,}1 \leq [A] \leq 10,$$

✧ de préférence

$$0{,}5 \leq [A] \leq 5,$$

✧ et plus préférentiellement encore

$$1,5 \leq [A] \leq 3,5.$$

S'agissant du substrat d'estérase chromogène (S), les quantités mises en oeuvre sont telles que sa concentration dans le milieu est définie comme suit (en mg/l) :

✧

$$50 \leq [\text{substrat}] \leq 1000,$$

✧ de préférence

$$100 \leq [\text{substrat}] \leq 800,$$

✧ et plus préférentiellement encore

$$200 \leq [\text{substrat}] \leq 600.$$

A propos du milieu de culture (M) présent dans le milieu détection/identification, il peut être précisé que l'on le sélectionne dans le groupe comprenant:

- les milieux séléctifs de type : Mac Conkey, Columbia ANC, PALCAM, Sabouraud gentamycine-chloramphénicol, de préférence le milieu Mac Conkey,
- les milieux non sélectifs de type Columbia +/- sang, Trypcase Soja, Gélose nutritive, Sabouraud, de préférence le milieu Columbia.

En pratique, l'homme de l'art choisira le milieu de culture (M) en fonction des microorganismes-cibles, selon des critères parfaitement connus et à la portée de cet homme de l'art.
Sans que cela ne soit limitatif, il s'avère que le milieu selon l'invention est particulièrement adapté à la détection/identification de bactéries du genre *Salmonella.* Dans ce cas, on choisira par exemple, le milieu de Mac Conkey à titre de milieu de culture.
Par ailleurs, le milieu selon l'invention peut contenir d'éventuels autres additifs comme par exemple : un ou plusieurs autres substrats enzymatiques par exemple chromogènes ou fluorogènes, des peptones, un ou plusieurs facteurs de croissance, des hydrates de carbone, un ou plusieurs agents sélectifs, des tampons, des gélifiants.
Le milieu selon l'invention se présente sous forme de liquide ou de gel prêt à l'emploi, c'est-à-dire prêt à l'ensemencement en tube, flacon ou sur des boîtes de Pétri.
Le milieu selon l'invention peut se conserver plusieurs semaines à 4°C dans ses conteneurs et sous forme liquide ou gel.
Selon un autre de ses aspects, la présente invention vise également un procédé d'obtention du milieu tel que défini ci-dessus caractérisé en ce qu'il consiste essentiellement :

✧ à préparer au moins une solution mère du substrat d'estérase chromogène ou fluorogène (S) dans le solvant C et d'au moins un agent solubilisant A,
✧ à ajouter cette solution au milieu de culture (M),
✧ à incorporer le détergent (B) et d'éventuels autres additifs au mélange S+C+A+M,

et à homogénéiser l'ensemble.
La préparation de la solution mère s'effectue de manière séparée en incorporant successivement le solvant (C), le substrat(S) et l'agent (A) - éventuellement co-additivé. Les produits et les quantités utilisés sont tels que définis ci-dessus.
Après homogénéisation, la solution mère est ajoutée au milieu de culture (M) gélifié mis en surfusion et préalablement régénéré dans l'eau. Il peut s'agir également d'un milieu liquide non gélifié, comme par exemple un bouillon nutritif.

Après mélange du milieu de culture (M) et de la solution mère, on obtient le milieu de détection liquide ou gélifié prêt à être ensemencé.

Selon encore un autre de ses aspects, l'invention a également pour objet un procédé de détection/identification de souches estérase+ (par exemple des salmonelles) consistant à :

o ensemencer le milieu de détection/identification tel que défini ci-dessus en tant que produit *per se* ou en tant que produit obtenu par le procédé également décrit ci-dessus, avec l'échantillon susceptible de contenir les micro-organismes à analyser ;
o incuber le milieu ensemencé dans des conditions appropriées connues de l'homme de l'art ;
o et lire/interpréter les colorations ou fluorescences des colonies qui se sont développées après incubation, par exemple à l'étuve à 37°C ; ces colorations ou fluorescences révélant l'hydrolyse du substrat d'estérase chromogène ou fluorogène par les micro-organismes cibles.

Enfin, l'invention concerne également l'utilisation dans une composition comportant un agent A tel que défini ci-dessus à titre d'activateur sélectif, d'au moins un produit (B) sélectionné dans le groupe comprenant:

■ les composés de formule (I):

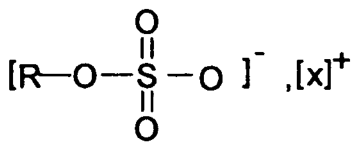

**(I)**

dans laquelle :

- R est un alkyle linéaire ou ramifié, de préférence en C 1-C30;
- X est un halogène, de préférence Na;

✧ les dérivés polyéthers non ioniques, de préférence ceux de formule (**II**):

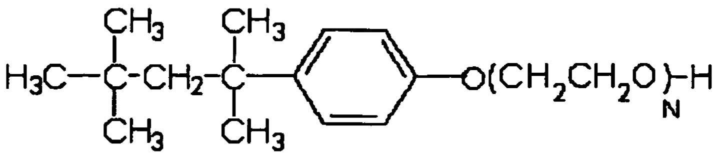

N est un nombre entier compris entre 2 et 15 (en moyenne = 10);
✧ et leurs mélanges.

Les exemples qui suivent permettront de mieux comprendre l'invention et d'appréhender tous ses avantages ainsi que ses diverses variantes de réalisation et de mise en oeuvre.

**EXEMPLES :**

**<u>Exemple 1 : utilisation du Tergitol 4 dans un milieu gélifié contenant un substrat d'estérase chromogénique pour améliorer l'expression de l'activité estérase des espèces bactériennes à Gram négatif présentant cette activité.</u>**

Le substrat d'estérase testé est le 5-Bromo-3-indolyl nonanoate. Une solution mère de substrat à 40 g/l est réalisée dans un mélange 40 % Diméthylsulfoxide et 60 % Tween® 20. Un volume suffisant est ajouté à un milieu Mac Conkey (sans rouge neutre) en surfusion pour obtenir une concentration en substrat de 500 mg/l.

Ce milieu est alors séparé en cinq milieux, dans chacun d'entre eux une concentration définie de Tergitol® 4 est ajoutée (voir tableau ci-dessous). Des micro-organismes issus de la collection de la Demanderesse ont été ensemencés sur ce milieu par isolement en trois quadrants à partir d'une suspension à 0,5 McFarland. Les boîtes ont été incubées à 37°C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 1 ci-dessous :

TABLEAU 1

| | Milieu | sans Tergitol 4 | | avec 2 ml/l de Tergitol 4 | | avec 4 ml/l de Tergitol 4 | | avec 8 ml/l de Tergitol 4 | | avec 16 ml/l de Tergitol 4 | |
|---|---|---|---|---|---|---|---|---|---|---|---|
| Souches | TI | C | I | C | I | C | I | C | I | C | I |
| *Salmonella spp.* 017 | 24 h | gris | 1 | gris | 1 | gris | **1,5** | gris | **2** | gris | 1 |
| | 48 h | gris | 2 | gris | 3 | gris | **3,5** | gris | 3 | gris | 2 |
| *Salmonella typhimurium* 010 | 24 h | gris | 2 | gris | 2 | gris | **3** | gris | **3,5** | gris | **3,5** |
| | 48 h | gris | 2 | gris | **2,5** | gris | **3** | gris | **3,5** | gris | **4** |
| *Salmonella enteritidis* 002 | 24 h | gris | 2 | gris | 2 | gris | **3** | gris | **3,5** | gris | **3,5** |
| | 48 h | gris | 2 | gris | 2 | gris | **3** | gris | **3,5** | gris | **3,5** |
| *Salmonella paratyphi A* 006 | 24 h | gris | 2 | gris | 2 | gris | 2 | gris | **3** | gris | 2 |
| | 48 h | gris | 2 | gris | 2 | gris | **2,5** | gris | **3** | gris | 2 |
| *Salmonella typhi* 118 | 24 h | gris | 2 | gris | 2 | gris | 2 | gris | **3** | gris | **3** |
| | 48 h | gris | 2 | gris | 2 | gris | **3** | gris | **3,5** | gris | **3,5** |
| *Salmonella arizonae* 018 | 24 h | gris | 2 | gris | 2 | gris | **3,5** | gris | **3,5** | gris | **3,5** |
| | 48 h | gris | 2,5 | gris | 2,5 | gris ; | **3,5** | gris ; | **3,5** | gris ; | **4** |
| *Escherichia coli* 002 | 24 h | - | - | - | - | - | - | - | - | - | - |
| | 48 h | - | - | - | - | - | - | - | - | - | - |
| *Hafnia alvei* 025 | 24 h | - | - | - | - | - | - | - | - | - | - |
| | 48 h | - | - | - | - | - | - | - | - | - | - |
| *Enterobacter cloacae* 086 | 24 h | gris | 0,5 | gris | 0,5 | gris | **1** | gris | 0,5 | gris | 0,5 |
| | 48 h | gris | 1,5 | gris | **2** | gris | **3** | gris | 1 | gris | 1 |
| *Serratia marcescens* 038 | 24 h | gris | 3 | gris | 2 | gris ; | 2 | gris | 2 | gris | 2 |
| | 48 h | gris | 3,5 | gris | 3 | gris | **4** | gris | **4** | gris | **4** |
| *Pseudomonas aeruginosa* 165 | 24 h | gris | 3 | gris | **3,5** | gris | **4** | gris | **4** | gris | **4** |
| | 48 h | gris | 3 | gris | **3,5** | gris | **4** | gris | **4** | gris | **4** |
| *Acinetobacter calcoaceticus* 034 | 24 h | gris | 2 | gris | 2 | gris | **3** | gris | **3** | gris | 0,5 |
| | 48 h | gris | 3 | gris | 3 | gris | 3 | gris | **4** | gris | 0,5 |
| - : absence de couleur et d'intensité ; TI : temps d'incubation ; intensité de coloration : échelle arbitraire | | | | | | | | | | | |

A une concentration comprise entre 2 et 16 ml/l, et plus précisément entre 4 et 8ml/1, le Tergitol® 4 augmente de manière significative l'intensité de coloration de souches bactériennes présentant une activité estérase. La concentration la plus efficace dépend des souches et aussi de l'effet recherché. Pour certaines souches il semble même possible

d'augmenter encore cette concentration.

**Exemple 2 utilisation du Tergitol 4 dans un milieu gélifié contenant un substrat d'estérase à base d'Indoxyle dissous dans un mélange DMSO et Sels** Biliaires.

Le substrat d'estérase testé est le 5-Bromo-3-indolyl nonanoate. Une solution mère de substrat à 25 g/l est réalisé dans le méthanol additionné de 5 g/l de Sels Biliaires. Un volume suffisant est ajouté à un milieu Mac Conkey (sans rouge neutre) en surfusion pour obtenir une concentration en substrat de 500 mg/l.

Ce milieu est alors séparé en cinq milieux, dans chacun d'entre eux une concentration définie de Tergitol® 4 est ajoutée (voir tableau ci-dessous). Des micro-organismes issus de la collection de la Demanderesse ont été ensemencés sur ce milieu par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 2 ci-dessous :

TABLEAU 2

| | Milieu | sans Tergitol 4 | | Avec 2 ml/l de Tergitol 4 | | avec 4 ml/l de Tergitol 4 | | avec 8 ml/l de Tergitol 4 | | avec 16 ml/l de Tergitol 4 | |
|---|---|---|---|---|---|---|---|---|---|---|---|
| souches | TI | C | I | C | I | C | I | C | I | C | I |
| *Salmonella spp.* | 24 h | - | - | gris | **1** | gris | **1** | gris ; | **1** | gris | **1** |
| 017 | 48 h | gris | 1 | gris | **1,5** | gris | **1,5** | gris | **1,5** | gris | **1,5** |
| *Salmonella typhimurium* | 24 h | gris | 0,5 | gris | **2** | gris | **2** | gris | **3** | gris | **3** |
| 010 | 48 h | gris | 1 | gris | **2** | gris | **3** | gris | **3** | gris | **3,5** |
| *Salmonella enteritidis* | 24 h | gris | 0,5 | gris | **2** | gris | **3** | gris | 0,5 | gris | 0,1 |
| 002 | 48 h | gris | **1** | gris | **3** | gris | **3** | gris | **2** | gris | 1 |
| *Salmonella paratyphi A* | 24 h | - | - | gris | **0,1** | gris | **0,1** | - | - | - | - |
| 006 | 48 h | gris | 0,5 | gris | 0,5 | gris | **1** | - | - | - | - |
| *Salmonella typhi* | 24 h | - | - | - | - | - | - | - | - | - | - |
| 118 | 48h | - | - | - | - | - | - | - | - | - | - |
| *Salmonella arizonae* | 24 h | gris | 0,5 | gris | **2,5** | gris | **2** | gris ; | 0,5 | gris | 0,5 |
| 018 | 48 h | gris | 1,5 | gris | **3** | gris | **2,5** | gris | 1,5 | gris | 2 |
| *Escherichia coli* | 24 h | - | - | - | - | - | - | - | - | - | - |
| 002 | 48 h | - | - | - | - | - | - | - | - | - | - |
| *Hafnia alvei* | 24 h | - | - | - | - | - | - | - | - | - | - |
| 025 | 48 h | - | - | - | - | - | - | - | - | - | - |
| *Enterobacter cloacae* | 24 h | gris | 0,5 | gris | 0,5 | gris | **1** | gris | 0,5 | gris ; | 0,5 |
| 086 | 48 h | gris | 1,5 | gris | **2** | gris | **3** | gris | 1 | gris | 1 |
| *Serratia marcescens* | 24 h | gris | **1** | gris | **2** | gris | **2** | gris | **2** | gris | **2** |
| 038 | 48 h | gris | 1,5 | gris | **3** | gris | **4** | gris | **4** | gris | **4** |
| *Pseudomonas aeruginosa* | 24 h | gris | 1 | gris | **3,5** | gris | **4** | gris | **4** | gris | **4** |
| 165 | 48 h | gris | 1,5 | gris | **3,5** | gris | **4** | gris | **4** | gris | **4** |

(suite)

| | Milieu | sans Tergitol 4 | | Avec 2 ml/l de Tergitol 4 | | avec 4 ml/l de Tergitol 4 | | avec 8 ml/l de Tergitol 4 | | avec 16 ml/l de Tergitol 4 | |
|---|---|---|---|---|---|---|---|---|---|---|---|
| souches | TI | C | I | C | I | C | I | C | I | C | I |
| *Acinetobacter calcoaceticus* 034 | 24 h | gris | 2 | gris | 2 | gris | **3** | gris | **3** | gris | 0,5 |
| | 48 h | gris | 3 | gris | 3 | gris | **3** | gris | **4** | gris | 0,5 |
| - : absence de couleur et d'intensité ; TI : temps d'incubation ; C : couleur ; I : intensité<br>intensité de coloration : échelle arbitraire | | | | | | | | | | | |

A une concentration comprise entre 2 et 16 ml/l, et plus précisément entre 4 et 8ml/l, le Tergitol® 4 augmente de manière significative l'intensité de coloration de souches bactériennes présentant une activité estérase. La concentration la plus efficace dépend des souches. Ces résultats montrent bien que l'effet du Tergitol® 4 sur l'activité estérase ne dépend pas du mode d'utilisation du substrat.

**Exemple 3 : utilisation du Tergitol 4 dans un milieu gélifié contenant un substrat d'estérase chromogénique pour améliorer l'expression de l'activité estérase des espèces bactériennes à Gram positif et des levures présentant cette** activité.

Le substrat d'estérase testé est le 5-Bromo-4-chloro-3-indolyl nonanoate. Une solution mère de substrat à 40 g/l est réalisé dans un mélange 40 % Diméthylsulfoxide et 60 % Tween® 20. Un volume suffisant est ajouté à un milieu Mac Conkey (sans rouge neutre) en surfusion pour obtenir une concentration en substrat de 250 mg/l.

Ce milieu est alors séparé en six milieux, dans chacun d'entre eux une concentration définie de Tergito® 4 est ajoutée (voir tableau ci-dessous). Des micro-organismes issus de la collection de la Demanderesse ont été ensemencés sur ce milieu par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 3 ci-dessous :

TABLEAU 3

| | Milieu | sans Tergitol 4 | | Avec 0,001 ml/l de Tergitol 4 | | avec 0,01 ml/l de Tergitol 4 | | avec 0,1 ml/l de Tergitol 4 | | avec 1 ml/l de Tergitol 4 | | avec 10 ml/l de Tergitol 4 | |
|---|---|---|---|---|---|---|---|---|---|---|---|---|---|
| Souches | TI | C | I | C | I | C | I | C | I | C | I | C | I |
| *Listeria monocytogenes* 023 | 24 h | T | 2 | T | 2 | T | **2,5** | T | 2 | T | 2 | T | 2 |
| | 48 h | T | 2 | T | 2 | T | **3** | T | **3** | T | **3** | T | **3** |
| *Listeria monocytogenes* 079 | 24 h | T | 2 | T | 2 | T | **3** | T | 2 | T | 2 | T | 2 |
| | 48 h | T | 3 | T | 3 | T | 3 | T | 3 | T | 3 | T | 3 |
| *Listeria innocua* 029 | 24 h | T | 0,1 | T | 0,1 | T | 0,1 | T | 0,1 | T | 0,1 | T | 0,1 |
| | 48h | T | 2 | T | 2 | T | 2 | T | 2 | T | 2 | T | 2 |
| *Staphylococcus aureus* 276 | 24 h | T | 1 | T | **2** | T | **2** | T | **3** | T | **2** | T | **2** |
| | 48h | T | 3 | T | 2 | T | **3** | T | **3** | T | **3** | T | **3** |
| *Staphylococcus epidermidis* 009 | 24 h | T | 2 | T | 2 | T | **3** | T | 2 | T | 2 | T | 0,5 |
| | 48h | T | 2 | T | 2 | T | **3** | T | **3** | T | 2 | T | **3** |
| *Salmonella spp.* 017 | 24 h | T | 2 | T | 2 | T | 2 | T | 2 | T | 2 | T | 2 |
| | 48h | T | 3 | T | 3 | T | 3 | T | 3 | T | 3 | T | 3 |

(suite)

| | Milieu | sans Tergitol 4 | | Avec 0,001 ml/l de Tergitol 4 | | avec 0,01 ml/l de Tergitol 4 | | avec 0,1 ml/l de Tergitol 4 | | avec 1 ml/l de Tergitol 4 | | avec 10 ml/l de Tergitol 4 | |
|---|---|---|---|---|---|---|---|---|---|---|---|---|---|
| Souches | TI | C | I | C | I | C | I | C | I | C | I | C | I |
| *Salmonella paratyphi A* | 24 h | T | 0,1 | T | 0,1 | T | 0,1 | T | 0,1 | T | **0,5** | T | **0,5** |
| 006 | 48 h | T | 0,5 | T | 0,5 | T | **1** | T | 0,5 | T | **2** | T | **2** |
| *Pseudomonas aeruginosa* | 24 h | T | 0,1 | T | 0,1 | T | **0,5** | T | 0,1 | T | **0,5** | T | **0,5** |
| 165 | 48h | T | 0,5 | T | 0,5 | T | **1** | T | 0,5 | T | 0,5 | T | **1** |
| *Candida albicans* | 24 h | T | 0,1 | T | **0,5** | T | **0,5** | T | **0,5** | T | **0,5** | T | **0,5** |
| 033 | 48h | T | ;2,5 | T | **3** | T | **3** | T | **3** | T | **3** | T | **3** |
| *Candida tropicalis* | 24 h | T | 0,5 | T | **2** | T | **2** | T | **2** | T | 0,5 | T | 0,5 |
| 030 | 48h | T | 3 | T | **3** | T | **3** | T | **3** | T | 3 | T | 3 |
| -: absence de couleur et d'intensité ; TI : temps d'incubation ; intensité de coloration : échelle arbitraire. | | | | | | | | | | | | | |

Le choix de concentrations en Tergitol® 4 très inférieures à celles testées dans les exemples précédents a été dicté par le type de souches testées. En effet, il s'agit soit de bactéries à Gram positif soit de levures ; or, à des concentrations trop importantes en Tergitol 4 ce type de souches ne peut se développer sur un milieu de culture.

De la même façon que dans les exemples précédents, il est possible d'observer un effet positif du Tergitol® 4 sur l'expression de l'activité estérase des micro-organismes testés. Cet effet, est variable d'une espèce bactérienne à une autre. Le choix d'une concentration en Tergitol® 4 dépend aussi des espèces. De manière générale, les concentrations efficaces pour les bactéries à Gram positif et les levures sont très inférieures à celles utilisées pour les bactéries à Gram négatif. Cependant, il est possible aux concentrations testées d'observer un léger impact positif sur la coloration des bactéries à Gram négatif testées dans cet exemple.

**Exemple 4 : utilisation de deux autres tensioactifs, le Tergitol® et le Triton® X100 (tensioactif non ionique) dans un milieu gélifié contenant un substrat d'estérase chromogénique pour améliorer l'expression de l'activité estérase des espèces bactériennes présentant cette activité.**

Le substrat d'estérase testé est le 5-Bromo-3-indolyl nonanoate. Une solution mère de substrat à 40 g/l est réalisé dans un mélange 40 % Diméthylsulfoxide et 60 % Tween 20. Un volume suffisant est ajouté à un milieu Mac Conkey (sans rouge neutre) en surfusion pour obtenir une concentration en substrat de 500 mg/l.

Ce milieu est alors séparé en huit milieux. Le premier milieu est directement réparti en boîtes de Pétri, dans un deuxième milieu 8 ml/l de Tergitol® 4 est ajouté, dans les trois suivants le Tergitol® 8 ajouté aux concentrations 2, 4 et 8 ml/l, enfin les trois derniers contiennent du Triton® X100 à 8, 16 et 32 ml/l. Des micro-organismes issus de la collection de la Demanderesse ont été ensemencés sur ce milieu par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation. La coloration de ces colonies ainsi que l'intensité de cette coloration ont été notées. Les résultats sont présentés dans les tableaux 4 et 5 ci-dessous :

TABLEAU 4

| | Milieu | sans Tergitol 4 | | avec 8 ml/l de Tergitol 4 | | avec 2 ml/l de Tergitol 8 | | avec 4 ml/l de Tergitol 8 | | avec 8 ml/l de Tergitol 8 | |
|---|---|---|---|---|---|---|---|---|---|---|---|
| souches | TI | C | I | C | I | C | I | C | I | C | I |
| *Salmonella spp.* 017 | 24 h<br>48 h | gris<br>gris | 1<br>2 | Gris<br>Gris | **2,5**<br>**3,5** | Gris<br>Gris | 0,5<br>1,5 | gris<br>gris | 0,3<br>1 | -<br>gris | -<br>0,5 |

(suite)

| | Milieu | sans Tergitol 4 | | avec 8 ml/l de Tergitol 4 | | avec 2 ml/l de Tergitol 8 | | avec 4 ml/l de Tergitol 8 | | avec 8 ml/l de Tergitol 8 | |
|---|---|---|---|---|---|---|---|---|---|---|---|
| souches | TI | C | I | C | I | C | I | C | I | C | I |
| *Salmonella enteritidis* 006 | 24 h | gris | 2 | Gris | **3** | Gris | **3** | gris | **2,5** | gris | **3** |
| | 48 h | gris | 2 | Gris | **3** | Gris | **4** | gris | **3** | gris | **3,5** |
| *Salmonella typhi* 118 | 24 h | gris | 2 | Gris | **3** | Gris | 1,5 | gris | 1 | gris | 1 |
| | 48 h | gris | 2 | Gris | **3,5** | Gris | 2 | gris | 1,5 | gris | 1 |
| *Salmonella arizonae* 018 | 24 h | gris | 2 | Gris | **3,5** | Gris | 2 | gris | 2 | gris | 2 |
| | 48 h | gris | 2,5 | Gris | **3,5** | Gris | **4** | gris | 2,5 | gris | 2,5 |
| *Escherichia coli* 002 | 24 h | - | - | - | - | - | - | - | - | - | - |
| | 48 h | - | - | - | - | - | - | - | - | - | - |
| *Hafnia alvei* 025 | 24 h | - | - | - | - | - | - | - | - | - | - |
| | 48 h | - | - | - | - | - | - | - | - | - | - |

TABLEAU 5

| | Milieu | Sans Tergitol 4 | | avec 8 ml/l de Tergitol 4 | | avec 8 ml/l de Triton X100 | | avec 16 ml/l de Triton X100 | | avec 32 ml/l de Triton X100 | |
|---|---|---|---|---|---|---|---|---|---|---|---|
| souches | TI | C | I | C | I | C | I | C | I | C | I |
| *Salmonella spp.* 017 | 24 h | gris | 1 | gris | **2,5** | gris | 1 | Gris | 0,5 | - | - |
| | 48 h | gris | 2 | gris | **3,5** | gris | **3** | Gris | 2 | gris | 0,5 |
| *Salmonella enteritidis* 006 | 24 h | gris | 2 | gris | **3** | gris | 2 | Gris | 2 | gris | 1 |
| | 48 h | gris | 2 | gris | **3** | gris | **2,5** | Gris | **2,5** | gris | 1,5 |
| *Salmonella typhi* 118 | 24 h | gris | 2 | gris | **3** | gris | 1 | Gris | 1 | gris | 0,5 |
| | 48 h | gris | 2 | gris | **3,5** | gris | 1 | gris | 1 | gris | 0,5 |
| *Salmonella arizonae* 018 | 24 h | gris | 2 | gris | **3,5** | gris | 2 | gris | 2 | gris | 1 |
| | 48 h | gris | 2,5 | gris | **3,5** | gris | 2,5 | gris | 2,5 | gris | 2 |
| *Escherichia coli* 002 | 24 h | - | - | - | - | - | - | - | - | - | - |
| | 48 h | - | - | - | - | - | - | - | - | - | - |
| *Hafnia alvei* 025 | 24 h | - | - | - | - | - | - | - | - | - | - |
| | 48 h | - | - | - | - | - | - | - | - | - | - |
| - : absence de couleur et d'intensité ; TI : temps d'incubation ; C : couleur ; I : intensité. intensité de coloration : échelle arbitraire | | | | | | | | | | | |

Différentes gammes de concentrations ont été testées au préalable afin d'étudier l'effet de ces composés sur la croissance bactérienne. Les points les plus intéressants ont alors été retenus pour cet exemple.

Dans les conditions expérimentales testées, le Tergito® 8 et le Triton® X100 ne permettent pas d'obtenir un effet aussi marqué sur l'activité estérase que celui observé avec le Tergitol® 4.

Note : Dans les exemples ci-dessus, les numéros annexés aux souches correspondent au numéro de chaque souche référencée dans la collection de la Demanderesse. L'intensité de coloration correspond à une échelle arbitraire dont la signification est la suivante :

0 pas d'activité

0,1 trace de coloration

0,5 coloration très pâle

(suite)

| | |
|---|---|
| 1 | coloration nette de faible intensité |
| 2 | coloration franche d'intensité moyenne |
| 3 | coloration intense |
| 4 | coloration très intense |

## Revendications

1. Milieu de détection/identification de microorganismes à activité estérasique, du type de ceux comprenant notamment un milieu réactionnel (M) et au moins un substrat d'estérase chromogène ou fluorogène (S) ; cette détection/identification reposant essentiellement sur la mise en évidence de l'activité estérase,
**caractérisé en ce que** :

    ✧ il est sous forme stable liquide ou gel prêt à l'emploi ;
    ✧ il comporte :

        o -A- au moins un agent solubilisant et stabilisant choisi dans le groupe comprenant :

            o les Esters de Sorbitan et d'Acide(s) Gras (ESAG),
            o les sels biliaires,
            o et leurs mélanges;

        o -B- au moins un activateur sélectif sélectionné dans le groupe comprenant:

            ■ les composés de formule (**I**) :

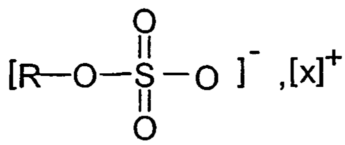

(I)

dans laquelle :

- R est un alkyle linéaire ou ramifié, de préférence en C1-C30;
- X est un halogène, de préférence Na;

✧ les dérivés polyéthers non ioniques, de préférence ceux de formule (**II**):

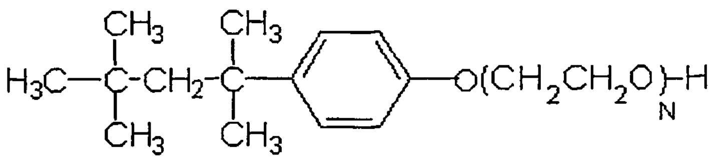

N est un nombre entier compris entre 2 et 15 (en moyenne = 10);
✧ et leurs mélanges;

o -C- et éventuellement au moins un Solvant.

2. Milieu selon la revendication 1 **caractérisé en ce qu'**il est obtenu par mélange d'au moins une solution mère de substrat(S) dans le solvant (C) et d'agent solubilisant (A) ajouté à au moins une partie des constituants du milieu

réactionnel (M) avec le promoteur (B).

**3.** Milieu selon la revendication 1 ou 2, **caractérisé en ce que** le promoteur sélectif (B) est un produit de formule (I) dans laquelle R correspond à un radical tétradécyle (Tergitol® ou Niaproof® 4) ou à un radical 2-éthylhexyle (Tergitol® ou Niaproof® 8).

**4.** Milieu selon la revendication 1 ou 2, **caractérisé en ce que** l'ESAG (A) est sélectionné dans le groupe comprenant :

✧ Monolaurate de Sorbitan polyoxyéthylèné comprenant 20 motifs oxyde d'éthylène (O.E), -TWEEN® 20-;
✧ Monopalmitate de Sorbitan polyoxyéthylèné (20 O.E), -TWEEN® 40-;
✧ Monostéarate de Sorbitan polyoxyéthylèné (20 O.E), -TWEEN® 60-;
✧ Tristéarate de Sorbitan polyoxyéthylèné (20 O.E), -TWEEN® 65-;
✧ Monooléate de Sorbitan polyoxyéthylèné (20 O.E), -TWEEN® 80-;
✧ Sesquioléate de Sorbitan polyoxyéthylèné (20 O.E), -TWEEN® 83-;
✧ Trioléate de Sorbitan polyoxyéthylèné (20 O.E), -TWEEN® 85-; et leurs mélanges.

**5.** Milieu selon la revendication 1, **caractérisé en ce que** les micro-organismes recherchés sont des bactéries gram$^+$ et/ou des levures et **en ce que** la concentration en agent B, exprimée en ml par litre de milieu (comprenant notamment: A, B, C, M, S) est telle que :

$$1.10^{-4} \leq [B] \leq 10,$$

de préférence

$$1.10^{-3} \leq [B] \leq 5,$$

et plus préférentiellement encore

$$1.10^{-2} \leq [B] \leq 2.$$

**6.** Milieu selon la revendication 1, **caractérisé en ce que** les micro-organismes recherchés sont des bactéries gram$^-$ et **en ce que** la concentration en agent B, exprimée en ml par litre de milieu (comprenant notamment: A, B, C, M, S) est telle que :

$$1.10^{-2} \leq [B] \leq 100,$$

de préférence

$$1.10^{-1} \leq [B] \leq 30,$$

et plus préférentiellement encore

$$1 \leq [B] \leq 25.$$

**7.** Milieu selon l'une quelconque des revendications 1 à 6 **caractérisé en ce que** le substrat d'estérase chromogène ou fluorogène est sélectionné dans le groupe comportant :

- les esters d'acide(s) gras et et d'Indoxyle et dérivés, de préférence les esters d'acide(s) gras en C6-C11 et d'Indoxyle, et plus préférentiellement encore les esters d'acide(s) gras en C8-C9 et d'Indoxyle éventuellement

halogénés;
- les esters d'acide(s) gras et de Quinone/Anthraquinone et dérivés, de préférence les esters d'acide(s) gras en C6-C11 et d'Anthraquinone, et plus préférentiellement encore les esters d'acide(s) gras en C8-C9 et de Dihydroxyanthraquinone (Alizarine);
- les esters d'acide(s) gras et d'Hydroxycoumarine et dérivés ;
- les esters d'acides gras et de Fluorescéine et dérivés ;
- et leurs mélanges.

8. Milieu selon l'une quelconque des revendications 1 à 7 **caractérisé en ce que** le solvant C est sélectionné dans le groupe comprenant :

■ les alcools, de préférence le Méthanol, l'Ethanol, le Méthoxyéthanol ;
■ les amides, de préférence le DiMéthylFormamide (DMF) ;
■ les solvants soufrés, de préférence le DiMéthylSulfOxyde (DMSO) ;
■ et leurs mélanges.

9. Milieu selon l'une quelconque des revendications 1 à 8 **caractérisé en ce que** la proportion pondérale agent (A) : solvant (C) est comprise entre 20 : 80 et 80 : 20, de préférence est comprise entre 30 : 70 et 70 : 30, et plus préférentiellement encore correspond à 40 : 60 ou à 60 : 40.

10. Milieu selon l'une quelconque des revendications 1 à 9 **caractérisé en ce que** la concentration en agent [A] dans le milieu est définie comme suit (en % en poids):

✧

$$0{,}1 \leq [A] \leq 10,$$

✧ de préférence

$$0{,}5 \leq [A] \leq 5,$$

✧ et plus préférentiellement encore

$$1{,}5 \leq [A] \leq 3{,}5.$$

11. Milieu selon l'une quelconque des revendications 1 à 10 **caractérisé en ce que** la concentration en substrat [S] dans le milieu est définie comme suit (en mg/l) :

✧

$$50 \leq [S] \leq 1000,$$

✧ de préférence

$$100 \leq [S] \leq 800,$$

✧ et plus préférentiellement encore

$$200 \leq [S] \leq 600.$$

**12.** Milieu selon l'une quelconque des revendications 1 à 11 **caractérisé en ce que** le milieu de culture (M) qu'il comprend est sélectionné dans le groupe comprenant:

- les milieux sélectifs de type Mac Conkey, Columbia ANC, PALCAM, Sabouraud Gentamycine-Chloramphénicol, de préférence le milieu Mac Conkey,
- les milieux non sélectifs de type Columbia +/- sang, Trypcase Soja, Gélose nutritive, Sabouraud, de préférence le milieu Columbia.

**13.** Milieu selon l'une quelconque des revendications 1 à 12 **caractérisé en ce que** les bactéries à détecter/identifier sont des salmonelles.

**14.** Procédé d'obtention du milieu selon l'une quelconque des revendications 1 à 13 **caractérisé en ce qu'**il consiste essentiellement :

✧ à préparer au moins une solution mère du substrat d'estérase chromogène ou fluorogène (S) dans le solvant C et d'au moins un agent solubilisant A,
✧ à ajouter cette solution au milieu réactionnel (M),
✧ à incorporer le détergent (B) et d'éventuels autres additifs au mélange S+A+C+M,
✧ et à homogénéiser l'ensemble.

**15.** Procédé de détection/identification de souches à activité estérasique **caractérisé en ce qu'**il consiste :

✧ à ensemencer le milieu selon l'une quelconque des revendications 1 à 13 ou le milieu obtenu par le procédé selon la revendication 14, avec l'échantillon susceptible de contenir les micro-organismes à analyser, détecter/identifier,
✧ à incuber le milieu ensemencé dans des conditions appropriées,
✧ et à lire et interpréter les colorations ou fluorescences des colonies, lesquelles colorations ou fluorescences révélant l'hydrolyse du substrat par les micro-organismes cibles.

**16.** Utilisation dans une composition comportant un agent A tel que défini dans la revendication 1 et éventuellement un solvant, à titre d'activateur sélectif, à savoir à titre de composé favorisant la pénétration des substrats d'estérase chromogènes ou fluorogènes ou l'excrétion d'enzymes au travers des membranes des cellules des microorganismes cibles, d'au moins un produit (B) sélectionné dans le groupe comprenant:

■ les composés de formule (I) :

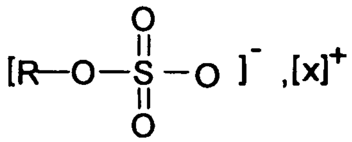

**(I)**

dans laquelle :

- R est un alkyle linéaire ou ramifié, de préférence en C1-C30;
- X est un halogène, de préférence Na;

✧ les dérivés polyéthers non ioniques, de préférence ceux de formule (II):

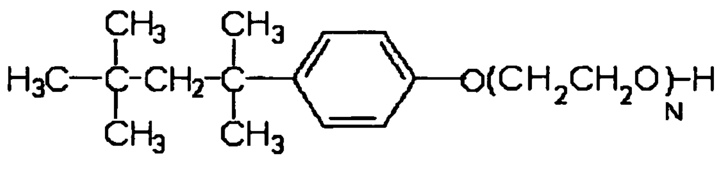

N est un nombre entier compris entre 2 et 15 (en moyenne = 10);
✧ et leurs mélanges.

## Claims

1. Medium for the detection/identification of microorganisms with esterase activity that is of the type comprising especially a reaction medium (M) and at least one chromogenic or fluorogenic esterase substrate (S), this detection/identification being based essentially on revealing the presence of the esterase activity, **characterized in that**:

   ✧ it is in a stable, ready-to-use liquid or gel form; and
   ✧ it contains:

   o -A- at least one solubilizer and stabilizer selected from the group comprising:

   o fatty acid sorbitan esters (FASE),
   o bile salts, and
   o mixtures thereof;

   o -B- at least one selective activator selected from the group comprising:

   ■ the compounds of formula (I):

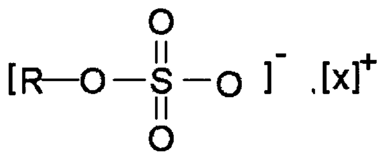

**(I)**

   in which:

   • R is a linear or branched alkyl, preferably C1-C30, and
   • X is a halogen, preferably Na;

   ■ non-ionic polyether derivatives, preferably those of formula (II):

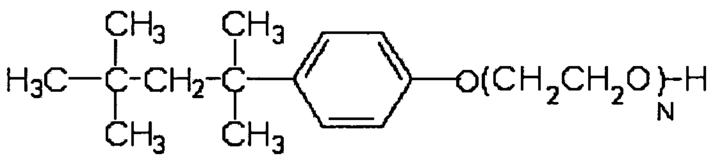

   in which: N is an integer between 2 and 15 (mean = 10); and
   ■ mixtures thereof; and

   o -C- optionally at least one solvent.

2. Medium according to claim 1, **characterized in that** it is obtained by mixing at least one stock solution of the substrate (S) and the solvent (C) with the solubilizer (A), added to at least some of the constituents of the reaction medium (M) with the promoter (B).

3. Medium according to claim 1 or 2, **characterized in that** the selective promoter (B) is a product of formula (I) in which R is a tetradecyl radical (Tergitol® or Niaproof® 4) or a 2-ethylhexyl radical (Tergitol® or Niaproof® 8).

4. Medium according to claim 1 or 2, **characterized in that** the FASE (A) is selected from the group comprising:

✧ polyethoxylated sorbitan monolaurate containing 20 units of ethylene oxide (EO) - TWEEN® 20-;
✧ polyethoxylated sorbitan monopalmitate (20 EO) - TWEEN® 40 -;
✧ polyethoxylated sorbitan monostearate (20 EO) - TWEEN® 60 -;
✧ polyethoxylated sorbitan tristearate (20 EO) - TWEEN® 65 -;
✧ polyethoxylated sorbitan monooleate (20 EO) - TWEEN® 80 -;
✧ polyethoxylated sorbitan sesquioleate (20 EO) - TWEEN® 83 -;
✧ polyethoxylated sorbitan trioleate (20 EO) - TWEEN® 85 -; and
✧ mixtures thereof.

5. Medium according to claim 1, **characterized in that** the target microorganisms are $Gram^+$ bacteria and/or yeasts and **in that** the concentration of agent (B), expressed in ml per liter of medium (comprising especially A, B, C, M and S), is such that:

$$1.10^{-4} \leq [B] \leq 10,$$

preferably

$$1.10^{-3} \leq [B] \leq 5,$$

and particularly preferably

$$1.10^{-2} \leq [B] \leq 2.$$

6. Medium according to claim 1, **characterized in that** the target microorganisms are $Gram^-$ bacteria and **in that** the concentration of agent (B), expressed in ml per liter of medium (comprising especially A, B, C, M and S), is such that:

$$1.10^{-2} \leq [B] \leq 100,$$

preferably

$$1.10^{-1} \leq [B] \leq 30,$$

and particularly preferably

$$1 \leq [B] \leq 25.$$

7. Medium according to any one of claims 1 to 6, **characterized in that** the chromogenic or fluorogenic esterase substrate is selected from the group comprising:

- indoxyl esters of fatty acid(s) and derivatives thereof, preferably indoxyl esters of C6-C11 fatty acid(s) and particularly preferably indoxyl esters of C8-C9 fatty acid(s) that are optionally halogenated;
- quinone/anthraquinone esters of fatty acid(s) and derivatives thereof, preferably anthraquinone esters of C6-C11 fatty acid(s) and particularly preferably dihydroxyanthraquinone (alizarin) esters of C8-C9 fatty acid(s);
- hydroxycoumarin esters of fatty acid(s) and derivatives thereof;
- fluorescein esters of fatty acids and derivatives thereof; and

- mixtures thereof.

**8.** Medium according to any one of claims 1 to 7, **characterized in that** the solvent (C) is selected from the group comprising:

- ■ alcohols, preferably methanol, ethanol and methoxyethanol;
- ■ amides, preferably dimethylformamide (DMF),
- ■ sulfur-containing solvents, preferably dimethyl sulfoxide (DMSO); and
- ■ mixtures thereof.

**9.** Medium according to any one of claims 1 to 8, **characterized in that** the proportion by weight of agent (A) to solvent (C) is between 20:80 and 80:20, preferably between 30:70 and 70:30 and particularly preferably 40:60 or 60:40.

**10.** Medium according to any one of claims 1 to 9, **characterized in that** the concentration of agent (A) in the medium is defined as follows (in % by weight):

- ✧

$$0.1 \leq [A] \leq 10,$$

- ✧ preferably

$$0.5 \leq [A] \leq 5,$$

- ✧ and particularly preferably

$$1.5 \leq [A] \leq 3.5.$$

**11.** Medium according to any one of claims 1 to 10, **characterized in that** the concentration of substrate (S) in the medium is defined as follows (in mg/1):

- ✧

$$50 \leq [S] \leq 1000,$$

- ✧ preferably

$$100 \leq [S] \leq 800,$$

- ✧ and particularly preferably

$$200 \leq [S] \leq 600.$$

**12.** Medium according to any one of claims 1 to 11, **characterized in that** the culture medium (M) it contains is selected from the group comprising:

- selective media of the type comprising MacConkey, Columbia ANC, PALCAM and Sabouraud gentamycin-chloramphenicol media, preferably MacConkey medium; and
- non-selective media of the type comprising Columbia +/- blood, trypcase soya, nutrient gelose and Sabouraud media, preferably Columbia medium.

13. Medium according to any one of claims 1 to 12, **characterized in that** the bacteria to be detected/identified are salmonellae.

14. Method of obtaining the medium according to any one of claims 1 to 13, **characterized in that** it consists essentially in:

✧ preparing at least one stock solution of the chromogenic or fluorogenic esterase substrate (S) in the solvent (C) and of at least one solubilizer (A),
✧ adding this solution to the reaction medium (M),
✧ incorporating the detergent (B) and any other additives into the S+A+C+M mixture, and
✧ homogenizing the whole.

15. Method for the detection/identification of strains with esterase activity, **characterized in that** it consists in:

✧ inoculating the medium according to any one of claims 1 to 13, or the medium obtained by the method according to claim 14, with the sample suspected of containing the microorganisms to be analyzed or detected/identified,
✧ incubating the inoculated medium under appropriate conditions, and
✧ reading and interpreting the colorations or fluorescences of the colonies, these colorations or fluorescences revealing hydrolysis of the substrate by the target microorganisms.

16. Use, in a composition comprising an agent (A) as defined in claim 1, and optionally a solvent, as a selective activator, that means as a compound which favors the penetration of the chromogenic or fluorogenic esterase substrates or the excretion of enzymes across the membranes of the cells of the target microorganisms, of at least one product (B) selected from the group comprising:

■ the compounds of formula (1):

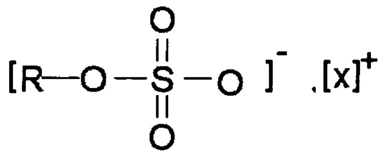

**(I)**

in which:

• R is a linear or branched alkyl, preferably C1-C30, and
• X is a halogen, preferably Na;

■ non-ionic polyether derivatives, preferably those of formula (II):

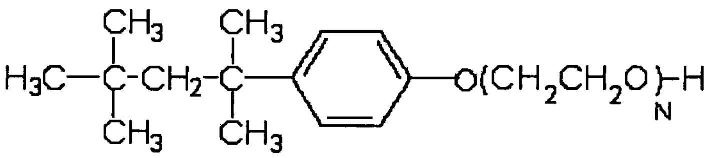

in which: N is an integer between 2 and 15 (mean = 10); and
■ mixtures thereof.

## Patentansprüche

1. Medium zur Detektion/Identifizierung von Mikroorganismen mit Esteraseaktivität, welches insbesondere die Form hat, dass es ein Reaktionsmedium (M) und mindestens ein chromogenes oder fluorogenes Esterasesubstrat (S)

umfaßt; wobei diese Detektion/Identifizierung im wesentlichen auf dem Nachweis der Esteraseaktivität beruht, **dadurch gekennzeichnet, daß**

✦ es in Form einer stabilen Flüssigkeit oder eines gebrauchsfertigen Gels vorliegt;
✦ es umfaßt:

A) mindestens ein Mittel zum Lösen und Stabilisieren, ausgewählt aus der Gruppe umfassend:

- Sorbitanfettsäureester (ESAG),
- Gallensalze
- und ihre Gemische;

B) mindestens einen selektiven Aktivator ausgewählt aus der Gruppe umfassend:

■ Verbindungen der Formel (I):

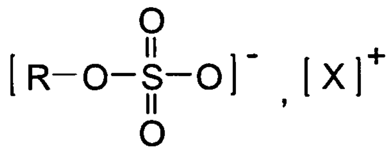

(I)

worin:

. R ein lineares oder verzweigtes, vorzugsweise $C_{1-30}$- Alkyl ist;
. X Halogen ist, vorzugsweise Na;

✦ die nicht-ionischen Polyetherderivate vorzugsweise solche der Formel (II) sind:

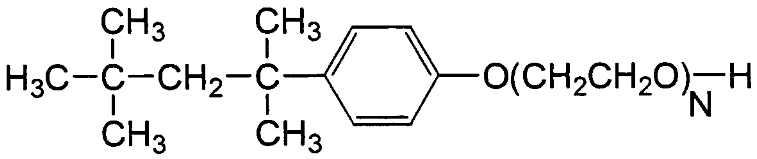

(II)

N eine ganze Zahl ist, die zwischen 2 und 15 liegt (Durchschnitt = 10);
✦ und deren Gemische;

C) gegebenenfalls mindestens ein Lösungsmittel.

2. Medium gemäß Anspruch 1, **dadurch gekennzeichnet, daß** es durch Mischen mindestens einer Ausgangslösung des Substrats (S) in dem Lösungsmittel (C) und dem Mittel zum Lösen (A), welche zu mindestens einem Teil der Bestandteile des Reaktionsmediums (M) mit dem Aktivator (B) gegeben werden, erhalten wird.

3. Medium gemäß Anspruch 1 oder 2, **dadurch gekennzeichnet, daß** der selektive Aktivator (B) ein Produkt der Formel (I) ist, worin R ein Tetradecylrest (Tergitol® oder Niaproof® 4) oder ein 2-Ethylhexylrest (Tergitol® oder Niaproof® 8) ist.

4. Medium gemäß Anspruch 1 oder 2, **dadurch gekennzeichnet, daß** der ESAG (A) ausgewählt ist aus der Gruppe umfassend:

· Polyoxyethylen-sorbitanmonolaurat umfassend 20 Ethylenoxid-(O.E.)-reste, -Tween® 20-,
· Polyoxyethylen-(20 O.E.)-sorbitanmonopalmitat, -Tween® 40-,
· Polyoxyethylen-(20 O.E.)-sorbitanmonostearat, -Tween® 60-,
· Polyoxyethylen-(20 O.E.)-sorbitantristearat, -Tween® 65-,

· Polyoxyethylen-(20 O.E.)-sorbitanmonooleat, -Tween® 80-,
· Polyoxyethylen-(20 O.E.)-sorbitansesquioleat, -Tween® 83-,
· Polyoxyethylen-(20 O.E.)-sorbitantrioleat, -Tween® 85-,

und deren Gemische.

**5.** Medium gemäß Anspruch 1, **dadurch gekennzeichnet, daß** die gesuchten Mikroorganismen Gram$^+$-Bakterien und/oder Hefen sind und **dadurch**, daß die Konzentration des Mittels B, in ml pro Liter Medium ausgedrückt (umfassend insbesondere: A, B, C, M, S), wie folgt ist:

$$1 \times 10^{-4} \leq [B] \leq 10$$

vorzugsweise

$$1 \times 10^{-3} \leq [B] \leq 5$$

mehr bevorzugt

$$1 \times 10^{-2} \leq [B] \leq 2$$

**6.** Medium gemäß Anspruch 1, **dadurch gekennzeichnet, daß** die gesuchten Mikroorganismen Gram--Bakterien sind und daß die Konzentration des Mittels B, in ml pro Liter Medium ausgedrückt (umfassend insbesondere: A, B, C, M, S), wie folgt ist:

$$1 \times 10^{-2} \leq [B] \leq 100$$

vorzugsweise

$$1 \times 10^{-1} \leq [B] \leq 30$$

mehr bevorzugt

$$1 \leq [B] \leq 25$$

**7.** Medium gemäß irgendeinem der Ansprüche 1 bis 6, **dadurch gekennzeichnet, daß** das chromogene oder fluorogene Esterasesubstrat ausgewählt ist aus der Gruppe umfassend:

- Fettsäureester und Indoxyl und Derivate davon, vorzugsweise $C_6$-$_{11}$-Fettsäureester und Indoxyl und noch bevorzugter $C_8$-$_9$-Fettsäureester und Indoxyl, die gegebenenfalls halogeniert sind;
- Fettsäureester und Chinon/Anthrachinon und Derivate davon, vorzugsweise $C_{6-11}$-Fettsäureester und Anthrachinon und noch bevorzugter $C_{8-9}$-Fettsäureester und Dihydroxyanthrachinon (Alizarin);
- Fettsäureester und Hydroxycoumarin und Derivate;
- Fettsäureester und Fluoreszin und Derivate;
- und deren Gemische.

**8.** Medium gemäß irgendeinem der Ansprüche 1 bis 7, **dadurch gekennzeichnet, daß** das Lösungsmittel C ausgewählt ist aus der Gruppe umfassend:

- Alkohole, vorzugsweise Methanol, Ethanol, Methoxyethanol;
- Amide, vorzugsweise Dimethylformamid (DMF);
- schwefelhaltige Lösungsmittel, vorzugsweise Dimethylsulfoxid (DMSO);
- und deren Gemische.

**9.** Medium gemäß irgendeinem der Ansprüche 1 bis 8, **dadurch gekennzeichnet, daß** der gewichtsmäßige Anteil des Mittels (A):Lösungsmittel (C) zwischen 20:80 und 80:20 liegt, vorzugsweise zwischen 30:70 und 70:30 und noch bevorzugter zwischen 40:60 oder 60:40.

**10.** Medium gemäß irgendeinem der Ansprüche 1 bis 9, **dadurch gekennzeichnet, daß** die Konzentration des Mittels [A] in dem Medium wie folgt definiert wird (in Gew.%):

$$0,1 \leq [A] \leq 10$$

vorzugsweise

$$0,5 \leq [A] \leq 5$$

mehr bevorzugt

$$1,5 \leq [A] \leq 3,5$$

**11.** Medium gemäß irgendeinem der Ansprüche 1 bis 10, **dadurch gekennzeichnet, daß** die Konzentration des Substrats [S] in dem Medium wie folgt definiert wird (in mg/1):

$$50 \leq [S] \leq 1.000$$

vorzugsweise

$$100 \leq [S] \leq 800$$

mehr bevorzugt

$$200 \leq [S] \leq 600$$

**12.** Medium gemäß irgendeinem der Ansprüche 1 bis 11, **dadurch gekennzeichnet, daß** das Kulturmedium (M), welches es umfaßt, ausgewählt ist aus der Gruppe umfassend:

- Selektionsmedien vom Typ Mac Conkey, Columbia ANC, PALCAM, Sabouraud Gentamycin-Chloramphenicol, vorzugsweise das Mac Conkey-Medium,
- nicht-selektiven Medien vom Typ Columbia +/- Blut, Trypcase Soja, Nährgelose, Sabouraud, vorzugsweise das Columbia-Medium.

**13.** Medium gemäß irgendeinem der Ansprüche 1 bis 12, **dadurch gekennzeichnet, daß** die zu detektierenden/identifizierenden Bakterien Salmonellen sind.

**14.** Verfahren zur Herstellung von Medium gemäß irgendeinem der Ansprüche 1 bis 13, **dadurch gekennzeichnet, daß** es im wesentlichen besteht aus:

- Herstellen mindestens einer Ausgangslösung des chromogenen oder fluorogenen Esterasesubstrats (S) im Lösungsmittel C und mindestens eines Lösungsmittels A,
- Hinzufügen dieser Lösung zum Reaktionsmedium (M),
- Einschließen des Detergens (B) und gegebenenfalls anderer Zusatzstoffe zum Medium S+A+C+M,
- und aus dem Homogenisieren davon.

**15.** Verfahren zum Detektieren/Identifizieren von Stämmen mit Esteraseaktivität, **dadurch gekennzeichnet, daß** es besteht aus:

- Besäen des Mediums gemäß irgendeinem der Ansprüche 1 bis 13 oder des Mediums, das durch das Verfahren gemäß Anspruch 14 gewonnen wurde, mit einer Probe, die unter dem Verdacht steht, zu analysierende, detektierende/identifizierende Mikroorganismen zu enthalten,
- Inkubieren des besäten Mediums unter geeigneten Bedingungen,
- Ablesen und Interpretieren der Fluoreszenzfärbungen der Kolonien, wobei die Fluoreszenzfärbungen die Hydrolyse des Substrats durch die Zielmikroorganismen anzeigen.

**16.** Verwendung, in einer Zusammensetzung, die ein Mittel A umfaßt, welches in Anspruch 1 definiert worden ist, und gegebenenfalls ein Lösungsmittel als selektiven Aktivator, das heißt als Verbindung, die das Eindringen der chromogenen oder fluorogenen Esterasesubstrate oder die Ausscheidung von Enzymen über die Zellmembranen der Zielmikroorganismen fördert, von mindestens einem Produkt (B), ausgewählt aus der Gruppe umfassend:

• Verbindungen der Formel (I):

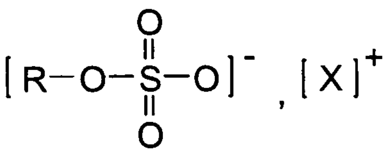

(I)

worin:

· R ein lineares oder verzweigtes Alkyl ist, vorzugsweise $C_{1-30}$;
· X Halogen ist, vorzugsweise Na;

✦ nicht-ionische Polyetherderivate, vorzugsweise solche der Formel (II):

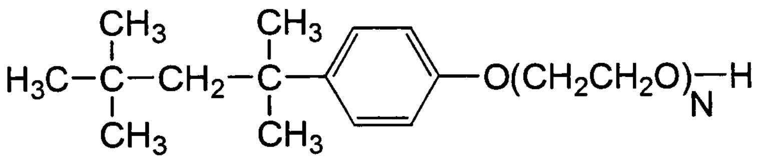

(II)

N ist eine ganze Zahl zwischen 2 und 15 (Durchschnitt = 10);
✦ und deren Gemische.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2697028
- WO 9217607 A
- WO 9941409 A